# EUROPEAN PATENT SPECIFICATION

(11) **EP 4 312 940 B1**
(45) Date of publication and mention of the grant of the patent: **26.02.2025**
(21) Application number: 22713991.2
(22) Date of filing: 17.03.2022
(51) Int. Cl.: A61H 23/00

(54) **SKIN TREATMENT APPARATUS**
HAUTBEHANDLUNGSVORRICHTUNG
APPAREIL DE TRAITEMENT DE LA PEAU

(30) Priority: 26.03.2021 GB 202104282
(43) Date of publication of application: 07.02.2024
(73) Proprietor: SCHOLL'S WELLNESS COMPANY LIMITED, London WC1N 3AX (GB)
(72) Inventor: LIU, Benjamin, Slough Berkshire SL1 3UH (GB); OLIVER, Richard, London WC1N 3AX (GB); SIMPSON, Adam, Slough Berkshire SL1 3UH (GB); WITTY, Christpher, Slough Berkshire SL1 3UH (GB)
(74) Representative: Marks & Clerk LLP
(86) International application number: PCT/GB2022/050674
(87) International publication number: WO 2022/200769

(56) References cited:
- WO-A1-2019/243844
- GB-A- 731 336
- US-A1- 2010 160 840

## Description

The present invention relates to a skin treatment device; and, more particularly, to an electrical skin treatment device for delivering more than one benefit with a roller head. The roller head may be used to remove a hard, dry skin layer on a surface of the foot, for example, the heel, the sole or the toes. The roller head may also be used for other skin treatments such as skin exfoliation, moisturising and/or massaging.

Calluses and/or areas of hard skin occur on feet and can be caused by persistent rubbing or uneven pressure, for example from ill-fitting shoes. They are most commonly found at the heel, the ball of the foot and the sides of the toes. Calluses are often unsightly, and the thicker they are the more yellow they can look. With time, particularly thick calluses can become cracked and painful. There are a number of known methods for reducing or removing calluses and hard skin which include electrically operated foot file devices.

The known skin treatment devices provide a single benefit for each roller head. The user needs to change the roller head in order to receive more than one benefit from the skin treatment device. Document GB 731 336 discloses a massage device with rotating flaps.

The present invention aims to provide multiple benefits with a single roller head, therefore mitigating the need for a user to switch roller heads in order to receive more than one benefit.

According to the present invention there is provided a roller head as defined in claim 1 as appended hereto. According to a further aspect of the present invention there is provided a skin treatment device comprising a roller head and an electro-mechanical mechanism configured to drive the drum roller head as defined in claim 12 as appended hereto. Preferred features are set out in the dependent claims.

Aspects of the disclosure are also described in detail, by way of example only, with reference to the accompanying drawings, in which:
Embodiments will now be described with reference to the Figures.
Figure 1 shows a roller head having flaps extending radially;
Figure 2 shows a perspective view of the roller head of Figure 1;
Figure 3 shows an individual flap from the roller head of Figure 1; and
Figure 4 show a perspective view of a roller head.

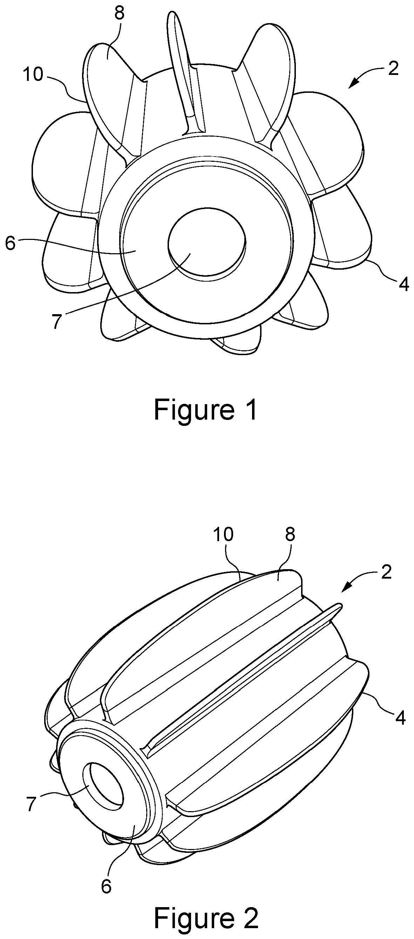

Figure 1 shows a roller head 2 for a skin treatment device (not shown).

The roller head 2 comprises a drum 6 and abrasive surface 4. The drum 6 comprises a shaft 7 at each end of the drum 6. The roller head 2 comprises one or more flaps 4 that extend radially from the drum. The one or more flaps comprise a first surface 8 and a second surface 10.

The first surface 8 differs from the second surface 10 so that the first surface 8 provides a different benefit to the user compared to the second surface 10. For example, rubbing the first surface 8 on a user's skin may provide a different benefit by having a different effect on the user's skin compared to rubbing the second surface 10 on the user's skin. This allows a single roller head to be used for more than one benefit.

The roller head is configured to be rotated by a skin treatment device. The skin treatment device couples to the roller head via shaft 7. This allows the skin treatment device to rotate the drum along its longitudinal axis. The rotation of the drum along its longitudinal axis allows a user to provide the benefit to the user's skin.

The one or more flaps 4 are configured to flex so that, in use, the first surface 8 of the flap 4 contacts with the skin with when the roller head rotated in a clockwise direction and a second surface 10 of the flap 4 contacts with the skin with when the roller head rotated in an anticlockwise direction. The user is therefore able to apply the first benefit from the first surface 8 and the second benefit from the second surface 10 by rotating the roller head in different directions along the longitudinal axis because the flap 4 is able to flex one way to allow the first surface 8 comes into contact with the user's skin in one direction and the second surface 10 comes into contact with the user's skin in another direction by the flap 4 flexing the other way. For example, when the roller head 2 is rotated in a clockwise direction the first surface 8 comes into contact with the user's skin and when the roller head 2 is rotated in an anticlockwise direction the second surface 10 comes into contact with the user's skin.

In the example shown, the flap comprises a flexible portion where the flap couples to the drum 6. The portion of the flap 4 that is proximate drum (i.e. at the base of the flap) is configured to flex. The flexible portion allows the flap to flex by rotating the flap about the flexible portion. The flexible portion of the flap is relatively more flexible than the remainder of the flap, for example the flexible portion is sufficiently flexible to allow the remainder of the flap to move about the flexible portion. The flexible portion may have a hardness in the Shore A range, for Shore 20A, 30A, 40A, 50A, 60A, 70A, 80A and/or 90A.

The benefit provided to the user's skin by the roller head may include removal of hard skin, skin smoothing, exfoliation, massaging, moisturisation and/or lubrication.

Figure 2 shows a perspective view of the roller head of Figure 1. Figure 2 show the flap in more detail. In this example, each flap is convex in shape, for example the flap is extends further in a radial direction towards the centre of the roller head relative to the end of the roller head.

Hard skin is removed using a roller head having a rough surface and the movement of the rough surface of the roller head on the skin removes the hard skin. The degree of roughness of surface of the roller head alters the rate at which hard skin is removed. For example, a roller head with a rougher surface will remove hard skin at a more quickly than a roller head with a less rough surface. A user may want to initially use a roller head having rougher surface and then use a less rough roller head to achieve optimum hard skin removal. The examples set out below may provide a convenient method for a user to achieve the required level of hard skin removal.

In the example illustrated in Figures 1 and 2, the roller head comprises one or more flaps 4 and therefore comprises one or more first surfaces 8 having a first roughness and one or second surfaces 10 having a second roughness.

In use, the user applies the first benefit from the first surface 8 to their skin by rotating the roller head 2 in a clockwise direction. As the roller head 2 is rotated clockwise, the first surface 8 of each flap sequentially comes into contact with the user's skin. As the first surfaces 8 of the roller contacts the user's skin the first benefit is applied to the user's skin. When the roller head is rotated in an anti-clockwise direction the second surface 10 of each flap 4 sequentially comes into contact with the user's skin and the second benefit is applied to the user's skin.

The example in Figure 2 shows a roller with an abrasive surface on each flap. In this example, the first surface 8 has a different roughness to the second surface 10. In other examples, the roughness of the first surface 8 may be equal to the roughness of the second surface 10.

The roller head 2 may be used in conjunction with a skin treatment device having an electro mechanical mechanism. The roller head 2 may be inserted into the skin treatment device and may be coupled so that the electro mechanical mechanism of the skin treatment device rotates the roller head. In an example, the skin treatment device comprises two arms and the shaft 7 of the roller head is held by the two arms of the skin treatment device.

The skin treatment device is configured to rotate the roller head by transferring the rotation of the electro mechanical mechanism to the roller head via the shaft 7.

In the example above, the roller head comprises 9 flaps. The roller may also comprise the roller head comprises 3 to 15 flaps, for example 4 to 14 flaps, for example 5 to 13 flaps, for example 6 to 12 flaps, for example 7 to 11 flaps, for example 8 to 10 flaps.

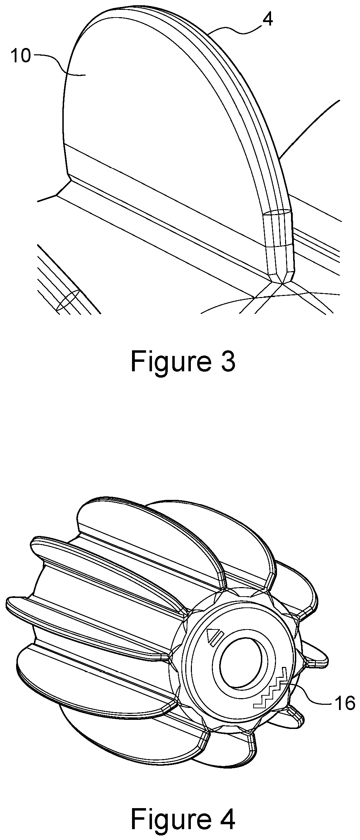

Figure 3 shows a single flap 4 showing the second surface 10 in detail. Figure 3 shows the convex shape of the flap and the second surface that is configured to come into contact with the skin when the roller head is rotated anti-clockwise.

In an example the flaps may comprise additional surface is located on the radial facing surface of the flap. The additional surface may be used to provide the user with the benefit of moisturisation, hard skin removal, lubrication, massaging, and/or exfoliation. For example, the first surface 8 may be used to remove hard skin, the second surface 10 may be used to smooth the hard skin and additional surface may be used to lubricate the skin.

In the examples illustrated above, the first surfaces 8 provide a first benefit and the second surfaces 10 provide a second benefit. The first surfaces 8 may provide more than one benefit and/or the second surfaces may provide more than one benefit. For example, the first surfaces and second surfaces may vary along the circumference of the roller head.

The additional portion, first surface and second surface may be used to different benefits to the user's skin. Two or more of the additional portion, first surface and second surface may also be used to provide the same benefit to the user's skin.

As illustrated in Figure 4, the roller head may have indicators 16 that show the user the orientation of the roller head required to achieve a first benefit and a second benefit. In an example, the roller head may have arrows, colour indications, writing and/or symbols on the surface that indicate the benefit provided by rotating the roller head in a given direction.

In the example described above the first surface of each flap provides a first benefit and the second surface of each flap provides a second benefit. In other examples, the roller head may comprise flaps with different first surfaces and different second surfaces.

In the examples described above, the flap is able to flex by rotating about a flexible portion. In other examples, the entire flap is flexible and the flex is configured to flex by the whole flap flexing. In this example the flap may be made from a single material and have a uniform flex. The flap may also comprise a hinge located at the base of the flap. The hinge would allow the flap to flex by rotating about the hinge i.e. a portion of the flap pivots about the hinge. The hinge may, for example, be formed from a metal pin. In an example, flap may comprise multiple hinges to allow the flap to rotate about multiple points along the radial length of the flap.

In an example, the flap may comprise a flexible portion located at the base of the flap and at least one additional flexible portion located in the radial direction of the flap to allow the flap to flex in more than one direction.

In the example described above, the flexible portion of the flap may be made from a soft rubber material, for example the flexible portion of the flap may be made from silicone, TPE, TPU, injection moulded foam and/or neoprene. In the example described above, the relatively less flexible portion of the flap may be made from ABS, PET, HDPE, PVC, LDPE and/or PP.

In an example the roller head may be manufactured using 3D printing. For example, the flaps may be formed using a 3D printer to forms the flexible part of the flap proximate the drum and the relatively less flexible part of the flap from a material that differs from the flexible part of the flap. The roller head may also be formed by injection moulding, moulding and/or casting. The roller head may also be form using a over moulding technique i.e. two shot moulding. In this example, the part of the flap is moulded and then the flexible portion of the flap over moulded onto the existing portion of the flap.

In the example described above, the additional surface is located of the radial facing surface of the flap. The additional surface may also be located radial end of the first surface and second surface of the one or more flaps.

Further modifications and developments can be made without departing from the scope of the invention defined in the appended claims.

## Claims

1. A roller head (2) for a skin treatment device;
the roller head comprises a drum and one or more flaps (4) that extend radially from the drum, and
the one or more flaps (4) comprises a first surface (8) and a second surface (10 and the one or more flaps (4) are configured to flex so that, in use, a first surface (8) of the flap (4) contacts the skin when the roller head (2) is rotated in a clockwise direction and a second surface (10) of the flap (4) contacts the skin when the roller head (2) is rotated in an anticlockwise direction,
**characterised in that**,
each flap (4) comprises a flexible portion at its base end coupled to the drum, and the flexible portion is more flexible than the remainder of the flap.

2. The roller head (2) of claim 1, wherein the first surface (8) differs from the second surface (10) and the first surface (8) is configured to provide a first benefit and the second surface (10) is configured to provide a second benefit.

3. The roller head (2) of claim 1, wherein the first surface (8) and/or second surface (10) is configured for removal of hard skin, skin smoothing, exfoliation, massaging, moisturisation and/or lubrication.

4. The roller head (2) of claim 2, wherein the roller head (2) is configured to remove hard skin and the first surface (8) has a first roughness and the second surface (10) has a second roughness.

5. The roller head (2) of any one of the preceding claims, further comprising an additional surface configured to provide a further benefit when the roller head (2) is rotated clockwise and anticlockwise.

6. The roller head (2) of claim 5, wherein the additional surface is configured to provide the further benefit of removal of hard skin, skin smoothing, exfoliation, massaging, moisturisation and/or lubrication.

7. The roller head (2) of claim 5, wherein the additional surface differs from the first surface and/or second surface.

8. The roller head (2) of claim 5, wherein the additional surface is configured to provide lubrication and/or moisturisation.

9. The roller head (2) of any of claims 5 to 8, wherein the additional surface is located on the radial facing surface of the flap (4).

10. The roller head (2) of any preceding claim, wherein the roller head (2) comprises 3 to 15 flaps (4), for example 4 to 14 flaps (4), for example 5 to 13 flaps (4), for example 6 to 12 flaps (4), for example 7 to 11 flaps (4).

11. The roller head (2) of any preceding claim, wherein the flap (4) is configured to flex by rotating about the flexible portion.

12. A skin treatment device comprising a roller head (2) and an electro-mechanical mechanism configured to drive the roller head (2);
the roller head (2) comprises a drum and one or more flaps (4) that extend radially from the drum;
the one or more flexible flaps (4) comprises a first surface (8) and a second surface (10) and the one or more flaps (4) are configured to flex so that, in use, a first surface (8) of the flap (4) contacts the skin when the roller head (2) is rotated in a clockwise direction and a second surface (10) of the flap (4) contacts the skin when the roller head (2) is rotated in an anticlockwise direction,
**characterised in that**,
each flap (4) comprises a flexible portion at its base end coupled to the drum, and the flexible portion is more flexible than the remainder of the flap.

13. The skin treatment device of claim 12, wherein the roller head (2) comprises any one of the features described in claim 2 to 12.

## Patentansprüche

1. Rollenkopf (2) für eine Hautbehandlungsvorrichtung;
der Rollenkopf umfasst eine Trommel und eine oder mehrere Klappen (4), die sich radial von der Trommel erstrecken,
und
die eine oder die mehreren Klappen (4) umfassen eine erste Oberfläche (8) und eine zweite Oberfläche (10), und die eine oder die mehreren Klappen (4) sind dazu konfiguriert, sich derart zu biegen, dass eine erste Oberfläche (8) der Klappe (4) bei Verwendung die Haut berührt, wenn der Rollenkopf (2) im Uhrzeigersinn gedreht wird, und eine zweite Oberfläche (10) der Klappe (4) die Haut berührt, wenn der Rollenkopf (2) gegen den Uhrzeigersinn gedreht wird,
**dadurch gekennzeichnet, dass**
jede Klappe (4) einen biegsamen Abschnitt an ihrem Basisende umfasst, der mit der Trommel gekoppelt ist, und der biegsame Abschnitt biegsamer ist als der Rest der Klappe.

2. Rollenkopf (2) nach Anspruch 1, wobei sich die erste Oberfläche (8) von der zweiten Oberfläche (10) unterscheidet, und die erste Oberfläche (8) dazu konfiguriert ist, einen ersten Nutzen bereitzustellen, und die zweite Oberfläche (10) dazu konfiguriert ist, einen zweiten Nutzen bereitzustellen.

3. Rollenkopf (2) nach Anspruch 1, wobei die erste Oberfläche (8) und/oder die zweite Oberfläche (10) zum Entfernen harter Haut, zum Glätten von Haut, zum Peeling, Massieren, Befeuchten und/oder Fetten konfiguriert ist.

4. Rollenkopf (2) nach Anspruch 2, wobei der Rollenkopf (2) dazu konfiguriert ist, harte Haut zu entfernen, und die erste Oberfläche (8) eine erste Rauheit aufweist, und die zweite Oberfläche (10) eine zweite Rauheit aufweist.

5. Rollenkopf (2) nach einem der vorstehenden Ansprüche, der ferner eine zusätzliche Oberfläche umfasst, die dazu konfiguriert ist, einen weiteren Nutzen bereitzustellen, wenn der Rollenkopf (2) im Uhrzeigersinn und gegen den Uhrzeigersinn gedreht wird.

6. Rollenkopf (2) nach Anspruch 5, wobei die zusätzliche Oberfläche zum Bereitstellen des weiteren Nutzens des Entfernens harter Haut, Glättens von Haut, Peelings, Massierens, Befeuchtens und/oder Fettens konfiguriert ist.

7. Rollenkopf (2) nach Anspruch 5, wobei sich die zusätzliche Oberfläche von der ersten Oberfläche und/oder der zweiten Oberfläche unterscheidet.

8. Rollenkopf (2) nach Anspruch 5, wobei die zusätzliche Oberfläche dazu konfiguriert ist, ein Fetten und/oder Befeuchten bereitzustellen.

9. Rollenkopf (2) nach einem der Ansprüche 5 bis 8, wobei sich die zusätzliche Oberfläche an der radial zugewandten Oberfläche der Klappe (4) befindet.

10. Rollenkopf (2) nach einem vorstehenden Anspruch, wobei der Rollenkopf (2) 3 bis 15 Klappen (4), beispielsweise 4 bis 14 Klappen (4), beispielsweise 5 bis 13 Klappen (4), beispielsweise 6 bis 12 Klappen (4), beispielsweise 7 bis 11 Klappen (4) umfasst.

11. Rollenkopf (2) nach einem vorstehenden Anspruch, wobei die Klappe (4) dazu konfiguriert ist, sich durch Drehen um den biegsamen Abschnitt zu biegen.

12. Hautbehandlungsvorrichtung, die einen Rollenkopf (2) und einen elektromechanischen Mechanismus, der dazu konfiguriert ist, den Rollenkopf (2) anzutreiben, umfasst;
der Rollenkopf (2) umfasst eine Trommel und eine oder mehrere Klappen (4), die sich radial von der Trommel erstrecken;
die eine oder die mehreren biegsamen Klappen (4) umfassen eine erste Oberfläche (8) und eine zweite Oberfläche (10), und die eine oder die mehreren Klappen (4) sind dazu konfiguriert, sich derart zu biegen, dass eine erste Oberfläche (8) der Klappe (4) bei Verwendung die Haut berührt, wenn der Rollenkopf (2) im Uhrzeigersinn gedreht wird, und eine zweite Oberfläche (10) der Klappe (4) die Haut berührt, wenn der Rollenkopf (2) gegen den Uhrzeigersinn gedreht wird,
**dadurch gekennzeichnet, dass**
jede Klappe (4) einen biegsamen Abschnitt an ihrem Basisende umfasst, der mit der Trommel gekoppelt ist, und der biegsame Abschnitt biegsamer ist als der Rest der Klappe.

13. Hautbehandlungsvorrichtung nach Anspruch 12, wobei der Rollenkopf (2) ein beliebiges der in Anspruch 2 bis 12 beschriebenen Merkmale umfasst.

## Revendications

1. Tête à rouleau (2) pour un dispositif de traitement de la peau ;
la tête à rouleau comprend un tambour et un ou plusieurs volets (4) qui s'étendent de manière radiale depuis le tambour,
et
les un ou plusieurs volets (4) comprennent une première surface (8) et une deuxième surface (10) et les un ou plusieurs volets (4) sont configurés pour fléchir de sorte que, pendant l'utilisation, une première surface (8) du volet (4) entre en contact avec la peau lorsque la tête à rouleau (2) est mise en rotation dans le sens des aiguilles d'une montre et une deuxième surface (10) du volet (4) entre en contact avec la peau lorsque la tête à rouleau (2) est mise en rotation dans le sens contraire aux aiguilles d'une montre,
**caractérisée en ce que**
chaque volet (4) comprend une partie flexible à son extrémité de base couplée au tambour, et la partie flexible est plus flexible que le reste du volet.

2. Tête à rouleau (2) selon la revendication 1, dans laquelle la première surface (8) diffère de la deuxième surface (10) et la première surface (8) est configurée pour fournir un premier avantage et la deuxième surface (10) est configurée pour fournir un deuxième avantage.

3. Tête à rouleau (2) selon la revendication 1, dans laquelle la première surface (8) et/ou la deuxième surface (10) est configurée pour une élimination de peau dure, un lissage de peau, une exfoliation, un massage, une humidification et/ou une lubrification.

4. Tête à rouleau (2) selon la revendication 2, dans laquelle la tête à rouleau (2) est configurée pour éliminer de la peau dure et la première surface (8) présente une première rugosité et la deuxième surface (10) présente une seconde rugosité.

5. Tête à rouleau (2) selon l'une quelconque des revendications précédentes, comprenant en outre une surface supplémentaire configurée pour fournir un avantage ultérieur lorsque la tête à rouleau (2) est tournée dans le sens des aiguilles d'une montre et dans le sens contraire aux aiguilles d'une montre.

6. Tête à rouleau (2) selon la revendication 5, dans laquelle la surface supplémentaire est configurée pour fournir l'avantage ultérieur d'élimination de peau dure, de lissage de peau, d'exfoliation, de massage, d'humidification et/ou de lubrification.

7. Tête à rouleau (2) selon la revendication 5, dans laquelle la surface supplémentaire diffère de la première surface et/ou de la deuxième surface.

8. Tête à rouleau (2) selon la revendication 5, dans laquelle la surface supplémentaire est configurée pour fournir une lubrification et/ou une humidification.

9. Tête à rouleau (2) selon l'une quelconque des revendications 5 à 8, dans laquelle la surface supplémentaire est située sur la surface qui fait face à la direction radiale du volet (4).

10. Tête à rouleau (2) selon l'une quelconque des revendications précédentes, dans laquelle la tête à rouleau (2) comprend 3 à 15 volets (4), par exemple 4 à 14 volets (4), par exemple 5 à 13 volets (4), par exemple 6 à 12 volets (4), par exemple 7 à 11 volets (4).

11. Tête à rouleau (2) selon l'une quelconque des revendications précédentes, dans laquelle le volet (4) est configuré pour fléchir en tournant autour de la partie flexible.

12. Dispositif de traitement de la peau comprenant une tête à rouleau (2) et un mécanisme électromécanique configuré pour entraîner la tête à rouleau (2) ;
la tête à rouleau (2) comprend un tambour et un ou plusieurs volets (4) qui s'étendent de manière radiale depuis le tambour ;
les un ou plusieurs volets (4) comprennent une première surface (8) et une deuxième surface (10) et les un ou plusieurs volets (4) sont configurés pour fléchir de sorte que, pendant l'utilisation, une première surface (8) du volet (4) entre en contact avec la peau lorsque la tête à rouleau (2) est mise en rotation dans le sens des aiguilles d'une montre et une deuxième surface (10) du volet (4) entre en contact avec la peau lorsque la tête à rouleau (2) est mise en rotation dans le sens contraire aux aiguilles d'une montre,
**caractérisé en ce que**
chaque volet (4) comprend une partie flexible à son extrémité de base couplée au tambour, et la partie flexible est plus flexible que le reste du volet.

13. Dispositif de traitement de la peau selon la revendication 12, dans lequel la tête à rouleau (2) comprend l'une quelconque des caractéristiques décrites dans les revendications 2 à 12.
